# EUROPÄISCHE PATENTSCHRIFT

(11) **EP 3 727 212 B1**
(45) Veröffentlichungstag und Bekanntmachung des Hinweises auf die Patenterteilung: **05.06.2024**
(21) Anmeldenummer: 18826306.5
(22) Anmeldetag: 18.12.2018
(51) Int. Cl.: A61F 2/70, A61F 2/68

(54) **VERSORGUNGSSYSTEM FÜR ORTHOPÄDIETECHNISCHE KOMPONENTE UND VERFAHREN**
SUPPLY SYSTEM FOR ORTHOPAEDIC TECHNOLOGY COMPONENTS AND METHOD
SYSTÈME D'ALIMENTATION POUR COMPOSANT ORTHOPÉDIQUE ET PROCÉDÉ

(30) Priorität: 22.12.2017 DE 102017131195
(43) Veröffentlichungstag der Anmeldung: 28.10.2020
(73) Patentinhaber: Otto Bock Healthcare Products GmbH, 1110 Wien (AT)
(72) Erfinder: PAPPE, Alexander, 1180 Wien (AT); WEIGL-POLLACK, Andreas, 3464 Goldgeben (AT); NOLTE, Michael, 37136 Seeburg (DE); ALBRECHT-LAATSCH, Erik, 37124 Rosdorf (DE); KAITAN, Robert, 1220 Wien (AT); HOFFMANN, Robert, 1160 Wien (AT); PAUSER, Thomas, 7035 Steinbrunn (AT); SCHRAMEL, Andreas, 1160 Wien (AT); SAGMEISTER, Luis, 2823 Pitten (AT)
(74) Vertreter: Gramm, Lins & Partner Patent- und Rechtsanwälte PartGmbB
(86) Internationale Anmeldenummer: PCT/EP2018/085663
(87) Internationale Veröffentlichungsnummer: WO 2019/121792

(56) Entgegenhaltungen:
- EP-A1- 2 842 521
- WO-A1-2010/064063
- WO-A2-2006/135851
- US-A1- 2015 351 941
- US-A1- 2016 250 045

## Beschreibung

Die Erfindung betrifft ein Versorgungssystem für zumindest eine orthopädietechnische Komponente, die zumindest eine elektronische und/oder elektrische Einrichtung aufweist, ein System aus einem solchen Versorgungssystem und einer orthopädietechnischen Komponenten und einem Verfahren zur Versorgung der orthopädietechnischen Komponente.

Orthopädietechnische Komponenten können insbesondere Prothesen, Orthesen, Rollstühle oder Teile von Prothesen, Orthesen oder Rollstühlen sein, beispielsweise Orthesen- oder Prothesengelenke, Rohradapter, Prothesenhände, Drehadapter, Prothesenfüße, Prothesenschäfte, Orthesenschienen, Orthesengelenke oder Aufnahmeschalen für einen Fuß sowie Komponenten von Rollstühlen oder auch Datenlogger, Funkmodule, Sensoren, Feedbackelemente oder Speichereinrichtungen zum Speichern elektrischer Energie.

Die Ausstattung orthopädietechnischer Komponenten mit elektrischen und/oder elektronischen Einrichtungen hat im Laufe der Entwicklung zu verbesserten, an den Patienten adaptierten oder adaptierbaren Einrichtungen geführt und zugenommen. Computergesteuerte Prothesen, Orthesen oder andere orthopädietechnische Komponenten sind zunehmend mit Antrieben ausgestattet. Ebenfalls können Dämpfereinrichtungen oder andere Aktuatoren oder Verstelleinrichtungen vorhanden sein, um eine Anpassung an den jeweiligen Patienten, die Umgebungsbedingungen und/oder gegenwärtige Bewegungssituationen vornehmen zu können. Dazu werden häufig Sensoren eingesetzt, über die Zustandsinformationen des Patienten, der Umgebung oder der orthopädietechnischen Komponente erfasst werden. Auf der Grundlage der Sensordaten werden dann über abgelegte Programme vorbestimmte Veränderungen vorgenommen, Kennlinien verändert, Antriebe aktiviert oder deaktiviert oder Gelenke freigegeben oder gesperrt.

Um die jeweilige orthopädietechnische Komponente dauerhaft betreiben zu können, sind häufig Energiespeicher oder Energieversorgungseinrichtungen sowie ein Steuerungsprogramm der orthopädietechnischen Komponente zugeordnet. Die Energiespeicher sind bevorzugt wiederaufladbar. Das Steuerungsprogramm sieht eine Schnittstelle zu Sensoren und externen Ausleseeinrichtungen oder Datenverarbeitungsgeräten vor, über die die ausgeführten Programme und/oder aufgenommenen Sensorwerte ausgewertet und gegebenenfalls Softwareaktualisierungen implementiert werden können.

Die WO 2010/064063 A1 befasst sich mit einer automatischen Prothese und einer elektronischen Vorrichtung zum Steuern dieser Prothese. Die Prothese weist einen USB-Anschluss auf, durch den Software in einen Datenspeicher und elektrische Energie übertragen werden. Ein Ladegerät kann über einen Magneten an der Prothese befestigt werden, um den Ladeschaltkreis zu erkennen

Die US 2015/351941 A1 befasst sich mit einer Prothese, die über einen kabellosen Anschluss verfügt, über den Informationen und elektrische Energie von einem externen Gerät auf die elektronische Steuerung innerhalb der Prothese übertragen werden können.

Die EP 2 842 521 A1 offenbart ein Prothesenvorrichtung mit einem prothetischen Knöchelgelenk, einem prothetischen Fuß und einem elektromagnetischen Empfänger. Der elektromagnetische Empfänger umfasst eine Vielzahl von Spulen, die nebeneinander angeordnet sind, so dass die Durchmesser der Spulen einen Abschnitt der Prothesenvorrichtung vollständig umgeben. Der elektromagnetische Empfänger ermöglicht ein drahtloses Aufladen.

Die Versorgung mit Energie und der Datenaustausch können im angelegten Zustand der orthopädietechnischen Komponente bei Orthesen oder Prothesen oder während der Benutzung beispielsweise von Rollstühlen erfolgen. Häufig erfolgt der Energieund Datenaustausch jedoch während Phasen der Nichtbenutzung.

Aufgabe der vorliegenden Erfindung ist es, den Daten- und/oder Energieaustausch oder allgemein die Wartung der orthopädietechnischen Komponente komfortabler zu gestalten.

Erfindungsgemäß wird diese Aufgabe durch ein Versorgungssystem, eine System und ein Verfahren mit den Merkmalen der unabhängigen Ansprüche gelöst. Vorteilhafte Ausgestaltungen und Weiterbildung der Erfindung sind in den Unteransprüchen, der Beschreibung sowie den Figuren offenbart.

Das erfindungsgemäße Versorgungssystem für zumindest eine orthopädietechnische Komponente, die zumindest eine elektronische und/oder elektrische Einrichtung aufweist und mit einem Versorgungsanschluss und/oder Funkeinrichtung zum Empfangen von Daten und/oder elektrischer Energie ausgestattet ist, und mit einer Halterung für die orthopädietechnische Komponente, die eine zu dem Versorgungsanschluss kompatible Versorgungseinrichtung zur Versorgung der orthopädietechnischen Komponente mit Daten und/oder Energie aufweist, wobei die Versorgungseinrichtung und der Versorgungsanschluss als kontaktlose Übertragungseinrichtung zur induktiven oder kapazitiven Übertragung oder zur Funkübertragung ausgebildet sind, sieht vor, dass das Versorgungssystem dazu eingerichtet ist, nach einer Koppelung der orthopädietechnischen Komponente mit der Versorgungseinrichtung eine Modusumschaltung der orthopädietechnischen Komponente von einem Betriebsmodus in einen Versorgungsmodus durchzuführen. Neben einer örtlichen Zuordnung der orthopädietechnischen Komponente, beispielsweise einer Orthese, einer Prothese oder eines Rollstuhls oder Teile einer Orthese, Prothese oder eines Rollstuhls, die als orthopädietechnische Komponente anzusehen sind, wird durch die Versorgungseinrichtung sichergestellt, dass während der Ruhephase oder während der Ablage der orthopädietechnischen Komponente an oder in der Halterung ein Datenaustausch und/oder Energieaustausch erfolgen kann, insbesondere eine Energiezufuhr zu einem Energiespeicher, der der orthopädietechnischen Komponente zugeordnet ist. Der Energiespeicher kann in der orthopädietechnischen Komponente integriert oder daran festgelegt sein. Ein Datenaustausch kann bidirektional erfolgen. Aufgenommene Daten der orthopädietechnischen Komponente können an die Versorgungseinrichtung übertragen werden, in der dann diese Daten ausgewertet werden können. Umgekehrt kann von der Versorgungseinrichtung ein Software-Update aufgespielt, eine Funktionsprüfung durchgeführt oder eine Fernwartung aktiviert werden, indem die Daten in die orthopädietechnische Komponente übertragen werden. Die orthopädietechnische Komponente weist dazu zumindest eine Speichereinrichtung für Daten sowie eine Verarbeitungseinrichtung oder einen Prozessor auf, um übertragene Daten zu speichern und zu verarbeiten.

Die elektronische und/oder elektrische Einrichtung kann einen elektrischen Verbraucher aufweisen, beispielsweise einen Antrieb zum Verstellen von Bauteilen der orthopädietechnischen Komponente zueinander. Darüber hinaus besteht die Möglichkeit, dass Sensordaten in einer elektronischen Einrichtung gespeichert und bei der Kopplung der orthopädietechnischen Komponente mit der Versorgungseinrichtung ausgelesen werden.

Die Halterung kann als Ablage oder Aufnahme mit zumindest einer Befestigungseinrichtung für die orthopädietechnische Komponente ausgebildet sein, über die die orthopädietechnische Komponente an der Halterung festlegbar ist. Neben einer Zuordnung der orthopädietechnischen Komponente zu der Versorgungseinrichtung wird darüber hinaus eine feste örtliche Zuordnung der orthopädietechnischen Komponente an der Halterung bewirkt, so dass die orthopädietechnische Komponente stets an der gleichen Stelle abgelegt und wieder aufgenommen werden kann. Insbesondere bei mehrgelenkigen Prothesen oder Orthesen wird durch die örtliche Festlegung der orthopädietechnischen Komponente zu der Halterung erreicht, dass auch während des Aufladens oder während der Versorgung der orthopädietechnischen Komponente im nicht angelegten Zustand die einzelnen Bauteile in einer definierten Zuordnung zueinander gehalten werden, so dass die orthopädietechnische Komponente nach dem Aufladen der Energiespeicher und der Aktualisierung der Software einfach wieder anlegbar ist.

Die Befestigungseinrichtung kann zumindest ein Formschlusselement und/oder ein Kraftschlusselement aufweisen, das mit einem korrespondierend ausgestatteten Formschlusselement oder Kraftschlusselement an der orthopädietechnischen Komponente wechselwirkt. Als Kraftschlusselemente sind insbesondere Magnete vorgesehen. Als formschlüssige Befestigungseinrichtung sind Vorsprünge, Hinterschneidungen, Klettverschlüsse, Klipsverschlüsse, Absätze oder korrespondierend zu der orthopädietechnischen Komponente ausgebildeten Aufnahmen vorgesehen. Bei einem sich konisch erweiternden Prothesenschaft kann die Befestigungseinrichtung eine trichterähnliche Aufnahme aufweisen. Durch die Befestigungseinrichtung wird sichergestellt, dass die orthopädietechnische Komponente während der Kopplung mit der Versorgungseinrichtung sicher an der Halterung gehalten bleibt.

Die Versorgungseinrichtung und der Versorgungsanschluss sind als kontaktlose Übertragungseinrichtungen ausgebildet, so dass beispielsweise bei einer kontaktlosen Übertragungseinrichtung Energie und Daten induktiv oder kapazitiv oder über Funk übertragen werden. Bei einer kontaktbehafteten Übertragung von Energie und/oder Daten ist eine Steckerverbindung vorgesehen, die auch selbstausrichtend ausgebildet sein kann. Die Steckerverbindung mit Stecker und Steckeraufnahme kann mit Formschlusselementen und/oder Kraftschlusselementen versehen sein, um eine Selbstausrichtung und somit eine Zentrierung von Stecker und Steckeraufnahme zueinander zu ermöglichen.

In der orthopädietechnischen Komponente kann eine Kodierung abgelegt sein, die über eine in oder an der Halterung angeordnete Identifikationseinrichtung auslesbar ist. Umgekehrt kann in der orthopädietechnischen Komponente eine solche Identifikationseinrichtung angeordnet sein, über die eine in oder an der Halterung angeordnete Kodierung auslesbar ist. Somit besteht die Möglichkeit zu einer gegenseitigen Authentifizierung von Halterung und orthopädietechnischer Komponente. Darüber hinaus können mehrere Teile einer orthopädietechnischen Komponente mit elektrischen und/oder elektronischen Einrichtungen ausgestattet sein, beispielsweise bei einer Beinprothese ein Prothesenkniegelenk und ein Prothesenknöchelgelenk oder bei einer Armprothese ein Prothesenellenbogengelenk sowie eine aktuierbare Prothesenhand. Jede dieser Teile einer orthopädietechnischen Komponente kann mit einer eigenständigen Kodierung ausgestattet sein, so dass alle Kodierungen von elektrischen und/oder elektronischen Komponenten von der Halterung auslesbar sind. Durch die gegenseitige Authentifizierung von Halterung und orthopädietechnischen Komponenten und deren Teile ist es möglich, den Umfang und die Art der übermittelten Energie bzw. der übermittelten Daten zu verändern. Dadurch ist es möglich, eine Halterung für mehrere orthopädietechnische Komponenten vorzusehen. Jede Halterung kann die ihr zugeordnete orthopädietechnische Komponente identifizieren und daran angepasst die notwendige Energiemenge, Energieart sowie Steuerungsprogramme, Daten oder ähnliches übermitteln oder auslesen.

An der Halterung kann eine Reinigungsvorrichtung angeordnet sein, um während des Datenaustausches oder der Energieversorgung die orthopädietechnische Komponente reinigen zu können und für den weiteren Gebrauch vorzubereiten. Die Reinigungsvorrichtung kann eine UV-Quelle, einen Plasmagenerator und/oder eine Röntgenröhre aufweisen, um durch eine entsprechende Bestrahlung oder Ionisierung die orthopädietechnische Komponente zu reinigen oder zu desinfizieren. Insbesondere bei unmittelbar auf der Haut getragenen orthopädietechnischen Komponenten ist eine solche integrierte Reinigungsvorrichtung vorteilhaft.

Die Erfindung betrifft ebenfalls ein System aus einem Versorgungssystem, wie es voranstehend beschrieben worden ist, und einer orthopädietechnischen Einrichtung, die über das Versorgungssystem mit Daten und/oder Energie versorgt werden kann. Die Ausgestaltung des Versorgungssystems und der orthopädietechnischen Einrichtung als Gesamtsystem erleichtert die gegenseitige Abstimmung und die reibungslose und störungsarme Versorgung mit Daten und/oder Energie.

Das Verfahren zur Versorgung einer orthopädietechnischen Komponente mit Daten und/oder Energie, wobei die orthopädietechnische Komponente zumindest eine elektronische und/oder elektrische Einrichtung aufweist und mit einem Versorgungsanschluss zum Empfangen von Daten und/oder elektrischer Energie ausgestattet ist, sieht vor, dass die orthopädietechnische Komponente in oder an einer Halterung, in oder an der eine zu dem Versorgunganschluss kompatible Versorgungseinrichtung zur Versorgung der orthopädietechnischen Komponente mit Daten und/oder Energie angeordnet ist, befestigt wird, dass der Versorgungsanschluss mit der Versorgungseinrichtung gekoppelt wird und automatisch nach der Kopplung der Versorgungseinrichtung mit dem Versorgungsanschluss eine Daten- und/oder Energieübertragung stattfindet. Sobald die orthopädietechnische Komponente in der Halterung abgelegt und auf der Halterung aufgelegt wird, finden eine Datenwartung und eine gegebenenfalls notwendige Energieübertragung statt. Vor der Daten- und/oder Energieübertragung kann eine Identifizierung der orthopädietechnischen Komponente erfolgen, beispielsweise durch Abfragen einer Kodierung der zumindest einen orthopädietechnischen Komponente oder aller orthopädietechnischen Komponenten oder Teile von orthopädietechnischen Komponenten mit mehreren elektronischen und/oder elektrischen Einrichtungen.

In Abhängigkeit von der orthopädietechnischen Komponente können dann abgestimmte Daten übermittelt und eine Funktionsüberprüfung eingeleitet werden. Bei einer Funktionsüberprüfung kann der komplette Funktionsumfang der orthopädietechnischen Komponente überprüft werden, beispielsweise eine maximale Verschwenkung von Elementen der orthopädietechnischen Komponente zueinander initiiert und überprüft werden, ob der maximale Verfahrweg und Verschwenkbereich erreicht wird. Auch können Verstellungen von Widerständen sowie andere Funktionsüberprüfungen während der Versorgung der orthopädietechnischen Komponente mit Daten und/oder Energie durchgeführt werden.

Nach der Kopplung der Versorgungseinrichtung mit dem Versorgungsanschluss der orthopädietechnischen Komponente erfolgt eine Modusumschaltung der orthopädietechnischen Komponente. Von einem Betriebsmodus wird nach der Kopplung mit der Versorgungseinrichtung in einen Versorgungsmodus umgeschaltet, bei dem einige Funktionen ausgeschaltet und andere aktiviert werden, um zu verhindern, dass die Funktionsüberprüfung im angelegten Zustand der orthopädietechnischen Komponente oder während deren eigentlichen Benutzung durchgeführt wird.

Im Versorgungsmodus können Zusatzfunktionen aktiviert werden, beispielsweise kann eine Musikwiedergabe stattfinden, um die Wartezeit zur Wiederinbetriebnahme der orthopädietechnischen Komponente möglichst angenehm zu gestalten.

Über eine gegenseitige Authentifizierung wird verhindert, dass falsche Daten übertragen werden oder dass sicherheitsrelevante Funktionen oder ein Quellcode ausgelesen und manipuliert werden.

Nachfolgend werden die Ausführungsbeispiele anhand der beigefügten Figuren näher erläutert. Es zeigen:
- Figur 1: eine erste Ausführungsform eines Versorgungssystems für eine Prothese einer unteren Extremität;
- Figur 2: eine zweite Ausführungsform des Versorgungssystems; sowie
- Figur 3: ein Versorgungssystem für eine Prothese einer oberen Extremität.

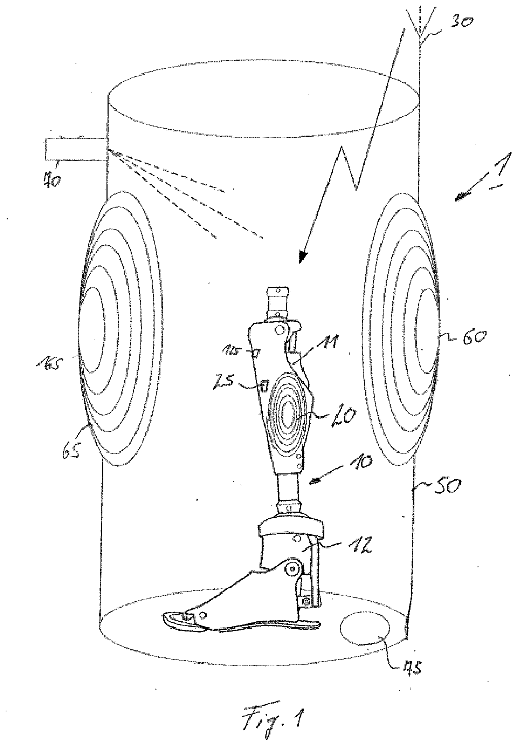

In der Figur 1 ist in einer schematischen Darstellung ein Versorgungssystem 1 für eine orthopädietechnische Komponente 10 in Gestalt einer Prothese für eine untere Extremität dargestellt. Die orthopädietechnische Komponente 10 weist ein Prothesenkniegelenk mit einer Unterschenkelkomponente, einem elektronisch gesteuerten Kniegelenk sowie einem Prothesenfuß auf. An der orthopädietechnischen Komponente 10 sind mehrere elektronische Einrichtungen 11, 12 angeordnet, die für die gewünschte Funktionsweise der orthopädietechnischen Komponente 10 wesentlich sind. In der Unterschenkelkomponente ist ein rechnergesteuerter Hydraulikaktuator angeordnet, dem neben Steuerungseinrichtungen Sensoren, Antriebe für Ventile oder Pumpen und Energiespeicher zugeordnet sind, über die eine Relativbewegung zwischen einem Oberteil, beispielsweise einem Anschluss für einen Prothesenschaft, und der Unterschenkelkomponente distal zu der Gelenkachse gesteuert wird. Die Bewegungswiderstände hinsichtlich der Extension und Flexion können über die elektronische Steuerung 11 verändert werden.

Weiterhin ist im Bereich des Knöchelgelenkes eine weitere elektrische und/oder elektronische Einrichtung 12 angeordnet, über die Flexions- und Extensionswiderstände des Fußteils relativ zu der Unterschenkelkomponente eingestellt werden können. Alternativ oder ergänzend sind Antriebe in den elektrischen und/oder elektronischen Einrichtungen 11, 12 angeordnet. An der orthopädietechnischen Komponente 10 ist ein Versorgungsanschluss 20 angeordnet, über den Daten und/oder elektrische Energie empfangen oder aufgenommen werden können, damit sowohl die Daten als auch die elektrische Energie innerhalb der orthopädietechnischen Komponente 10 gespeichert und verarbeitet werden können. In dem dargestellten Ausführungsbeispiel ist der Versorgungsanschluss 20 als ein drahtloser Versorgungsanschluss ausgebildet, beispielsweise eine Spuleneinrichtung, um unter Nutzung der Induktion Energie zu übertragen. Darüber hinaus kann eine Funkeinrichtung 31 zum Empfangen von Daten vorgesehen sein, um Informationen, beispielsweise ein Softwareupdate, aufzunehmen und zu speichern.

Ebenfalls ist an der orthopädietechnischen Komponente 10 eine Kodierung 25 abgelegt, über die die orthopädietechnische Komponente 10 eindeutig identifizierbar ist. Die Kodierung 25 kann in einem Transponder, Sender und/oder einer auslesbaren Speichereinrichtung abgelegt sein oder als Strichcode, QRC oder andere Markierung an der orthopädietechnischen Komponente 10 angeordnet sein. Die orthopädietechnische Komponente 10 befindet sich in einer Halterung 50, die in dem dargestellten Ausführungsbeispiel schematisch als eine zylindrische Halterung ausgebildet ist, die einen Boden aufweist, in dem eine Öffnung 75 als ein Auslass angeordnet ist. An der Halterung 50 ist eine Reinigungsvorrichtung 70 angeordnet, die als eine UV-Quelle, als ein Plasmagenerator, als eine Dusch- oder Wascheinrichtung oder als eine Röntgenröhre ausgebildet sein kann. Grundsätzlich ist es möglich, mehrere, auch unterschiedliche Reinigungsvorrichtungen 70 an einer Halterung 50 anzuordnen. Ist die Reinigungsvorrichtung 70 als eine Duscheinrichtung mit Pumpe und Sprühelement zum Versprühen von Reinigungsflüssigkeiten ausgebildet, kann überflüssige Reinigungsflüssigkeit über den Abfluss 75 ablaufen. Die orthopädietechnische Komponente 10 kann über eine obere Öffnung in die Halterung 50 eingestellt werden. Die obere Öffnung kann über einen Deckel verschließbar sein. Die Wand kann zumindest teilweise durchsichtig oder mit einem Fenster ausgestattet sein.

An der Halterung 50 ist eine Antenne 30 angeordnet, über die einerseits Daten empfangen und andererseits Daten zu der Funkeinrichtung 31 übermittelt werden können. Dies ist durch den Pfeil angedeutet. Die Antenne 30 ist Teil der Funkeinrichtung 30, 31 oder einer Versorgungseinrichtung 60, die an einer Außenwand der Halterung 50 und an der orthopädietechnischen Komponente 10 angeordnet sind, über die Daten über die Antenne 30 an die orthopädietechnische Komponenten 10 und die elektrischen und/oder elektronischen Einrichtungen 11, 12 übermittelt werden. Ebenfalls an der Halterung 50 ist eine Identifikationseinrichtung 65, 80 angeordnet, über die die Kodierung 25 der orthopädietechnischen Komponente 10 auslesbar ist. Die Kodierung 25 kann rein optisch erfasst und über eine Bildauswertung identifiziert werden. Alternativ oder ergänzend kann der Transponder mit der Kodierung 25 angeregt und ausgelesen werden. Ebenfalls kann ein Sender die Kodierung 25 permanent oder in Zeitabständen senden und das Signal von der Identifikationseinrichtung empfangen und ausgewertet werden.

Alternativ oder ergänzend kann an oder in der orthopädietechnischen Komponente 10 eine Identifikationseinrichtung 125 angeordnet oder integriert sein, über die eine in oder an der Halterung 50 angeordnete Kodierung 165 auslesbar ist. Über die jeweilige Identifikationseinrichtung 65, 125 und Kodierung 25, 165 ist es möglich, die jeweilige Halterung 50 auf die in ihr angeordnete orthopädietechnische Komponente 10 abzustimmen und die Art und Weise, wie Energie und Daten übertragen werden und insbesondere, welche Energiemengen und welche Arten von Daten übertragen werden, festzulegen und anzupassen.

In dem in der Figur 1 dargestellten Ausführungsbeispiel ist die orthopädietechnische Komponente 10 nur in der Halterung 50 abgestellt. An dem Boden der Halterung 50 kann beispielsweise eine Aufnahme oder ein Befestigungselement angeordnet sein, um die orthopädietechnische Komponente 10 in der Halterung 50 zu fixieren. Dies kann beispielsweise über Formschlusselemente, Klettverschlüsse, Schnappverschlüsse, Laschen, Riemen, Vorsprünge, Haken oder auch Magnete geschehen.

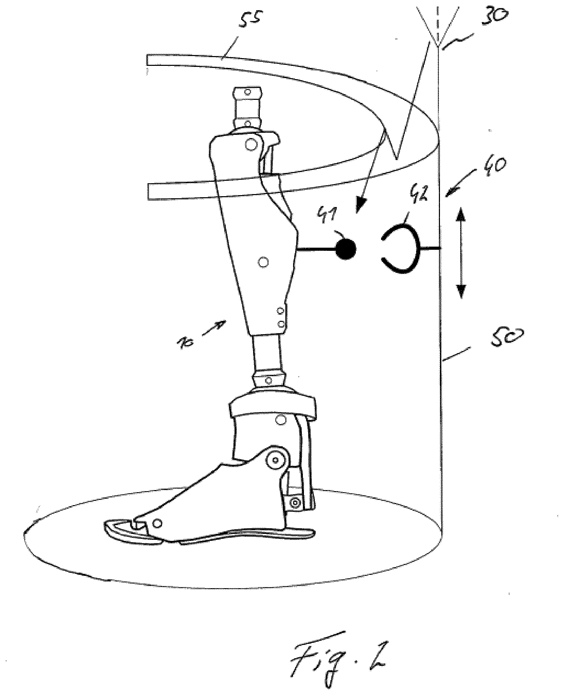

In der Figur 2 ist eine Variante der Halterung 50 gezeigt, die ebenfalls einen Boden und eine Seitenwand aufweist. An dem oberen Ende der Halterung 50 ist ein Rahmen angeordnet, um einen oberen Abschluss, beispielsweise zum Anlehnen oder zur Ablage der orthopädietechnischen Komponente 10 auszubilden. Anders als bei der Figur 1 ist in der Ausführungsform gemäß Figur 2 neben einer drahtlosen Datenübertragung über die Antenne 30 eine mechanische Kopplung über eine Befestigungseinrichtung 40 vorgesehen, bei der an der Halterung 50 eine erste Formschlusskomponente 42 höhenverstellbar angeordnet ist. Die Verstellbarkeit, insbesondere Verschieblichkeit ist über den Doppelpfeil angedeutet. Über die Höhenverlagerbarkeit ist es möglich, unterschiedliche orthopädietechnische Komponenten 10 in einer Halterung 50 aufzunehmen. An der orthopädietechnischen Komponente 10 ist ein korrespondierendes Formschlusselement 41 angeordnet, das neben einer mechanischen Verriegelung mit dem korrespondierenden Formschlusselement 42 und der dadurch verwirklichten Haltefunktion zur Energieübertragung ausgebildet ist. Das heißt, dass die beiden Formschlusselemente 41, 42 als Stecker und Steckaufnahme ausgebildet sind und somit gleichzeitig geben einer mechanischen Verriegelung auch eine elektrische Kontaktierung ausbilden. Neben einer reinen Energieübertragung kann zusätzlich auch eine Datenübertragung über die Kontaktierung mittels der Formschlusselemente 41, 42 erfolgen.

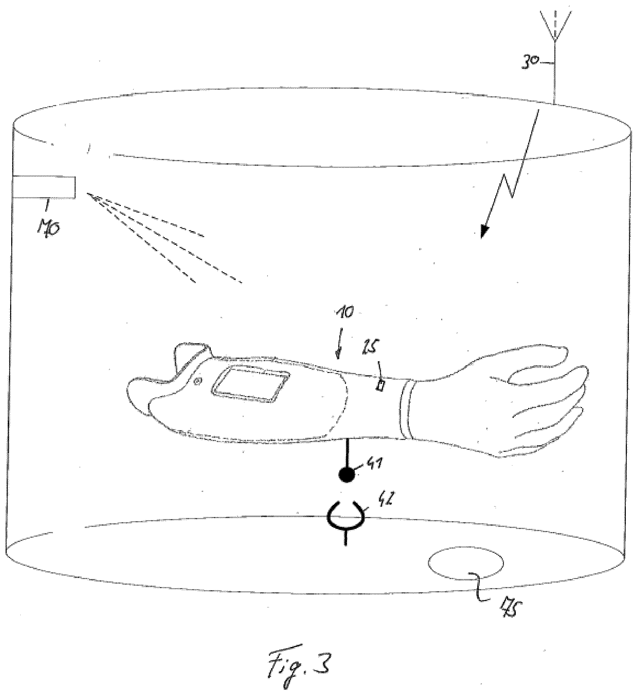

In der Figur 3 ist eine Variante der Figur 1 dargestellt, bei der eine Prothese für eine obere Extremität als orthopädietechnische Komponente 10 innerhalb der Halterung 50 angeordnet ist. Über die beiden Formschlusselemente 41, 42 wird die orthopädietechnische Komponente 10 in einer definierten Position innerhalb der Halterung 50 gehalten. Ebenfalls ist an der Halterung 50 eine Reinigungsvorrichtung 70 angeordnet, um nach dem Ablegen der orthopädietechnischen Komponente 10 diese reinigen zu können.

## Patentansprüche

1. Versorgungssystem für zumindest eine orthopädietechnische Komponente (10), die zumindest eine elektronische und/oder elektrische Einrichtung (11, 12) aufweist und mit einem Versorgungsanschluss (20) und/oder einer Funkeinrichtung (31) zum Empfangen von Daten und/oder elektrischer Energie ausgestattet ist, das Versorgungssystem hat eine Halterung (50) für die orthopädietechnische Komponente (10), die eine zu dem Versorgungsanschluss (20) und/oder der Funkeinrichtung (31) kompatible Versorgungseinrichtung (30, 60) zur Versorgung der orthopädietechnischen Komponente (10) mit Daten und/oder Energie aufweist, wobei die Versorgungseinrichtung (60) und der Versorgungsanschluss (20) als kontaktlose Übertragungseinrichtungen zur induktiven oder kapazitiven Übertragung oder zur Funkübertragung ausgebildet sind, **dadurch gekennzeichnet, dass** das Versorgungssystem dazu eingerichtet ist, nach einer Koppelung der orthopädietechnischen Komponente (10) mit der Versorgungseinrichtung (30, 60) eine Modusumschaltung der orthopädietechnischen Komponente (10) von einem Betriebsmodus in einen Versorgungsmodus durchzuführen.

2. Versorgungssystem nach Anspruch 1, **dadurch gekennzeichnet, dass** die Halterung (50) als Ablage oder Aufnahme mit zumindest einer Befestigungseinrichtung (40) ausgebildet ist, über die die orthopädietechnische Komponente (10) an der Halterung (50) festlegbar ist.

3. Versorgungssystem nach Anspruch 2, **dadurch gekennzeichnet, dass** die Befestigungseinrichtung (40) zumindest ein Formschlusselement (42) und/oder ein Kraftschlusselement aufweist, das mit einem korrespondierend ausgestatteten Formschlusselement (41) oder Kraftschlusselement an der orthopädietechnischen Komponente (10) wechselwirkt.

4. Versorgungssystem nach einem der voranstehenden Ansprüche, **dadurch gekennzeichnet, dass** in der orthopädietechnischen Komponente (10) eine Kodierung (25) abgelegt ist, die über eine in oder an der Halterung (50) angeordnete Identifikationseinrichtung (65, 80) auslesbar ist und/oder dass in der orthopädietechnischen Komponente (10) eine Identifikationseinrichtung (125) angeordnet ist, über die eine in oder an der Halterung (50) angeordnete Kodierung (165) auslesbar ist.

5. Versorgungssystem nach einem der voranstehenden Ansprüche, **dadurch gekennzeichnet, dass** an der Halterung (50) eine Reinigungsvorrichtung (70) angeordnet ist.

6. Versorgungssystem nach Anspruch 5, **dadurch gekennzeichnet, dass** die Reinigungsvorrichtung (70) eine UV-Quelle, einen Plasmagenerator und/oder eine Röntgenröhre aufweist.

7. System aus einem Versorgungssystem nach einem der voranstehenden Ansprüche und einer orthopädietechnischen Komponente.

8. Verfahren zur Versorgung einer orthopädietechnischen Komponente (10) mit Daten und/oder Energie, wobei die orthopädietechnische Komponente (10) zumindest eine elektronische und/oder elektrische Einrichtung (11, 12) aufweist und mit einem Versorgungsanschluss (20) und/oder einer Funkeinrichtung (31) zum Empfangen von Daten und/oder elektrischer Energie ausgestattet ist, wobei die orthopädietechnische Komponente (10) in oder an einer Halterung (50) und an einem Versorgungssystem nach einem der voranstehenden Ansprüche, in oder an der eine zu dem Versorgungsanschluss (20) und/oder Funkeinrichtung (31) kompatible Versorgungseinrichtung (30, 60) zur Versorgung der orthopädietechnischen Komponente (10) mit Daten und/oder Energie angeordnet ist, befestigt wird, der Versorgungsanschluss (209 und/oder die Funkeinrichtung (31) mit der Versorgungseinrichtung (30, 60) gekoppelt wird und automatisch eine Daten- und/oder Energieübertragung stattfindet, **dadurch gekennzeichnet, dass** nach der Koppelung mit der Versorgungseinrichtung (30, 60) eine Modusumschaltung der orthopädietechnischen Komponente (10) von einem Betriebsmodus in einen Versorgungsmodus durchgeführt wird.

9. Verfahren nach Anspruch 8, **dadurch gekennzeichnet, dass** vor der Datenund/oder Energieübertragung eine Identifizierung der orthopädietechnischen Komponente (10) erfolgt.

10. Verfahren nach Anspruch 9, **dadurch gekennzeichnet, dass** in Abhängigkeit von der orthopädietechnischen Komponente (10) auf die jeweilige orthopädietechnische Komponente (10) abgestimmte Daten übermittelt oder Funktionsüberprüfungen eingeleitet werden.

## Claims

1. A supply system for at least one orthopedic component (10) which comprises at least one electronic and/or electrical device (11, 12) and is equipped with a supply connection (20) and/or a radio device (31) for receiving data and/or electrical energy, the supply system having a holder (50) for the orthopedic component (10), which comprises a supply device (30, 60), compatible with the supply connection (20) and/or the radio device (31), for supplying the orthopedic component (10) with data and/or energy, whereas the supply device (60) and the supply connection (20) are configured as contactless transmission devices for inductive or capacitive transmission or radio transmission, **characterized in that** the supply system is configured to carry out a mode switchover of the orthopedic component (10) from an operating mode to a supply mode after the coupling of the orthopedic component (10) to the supply device (30, 60).

2. The supply system as claimed in claim 1, **characterized in that** the holder (50) is configured as a storage place or receptacle having at least one fastening device (40), by means of which the orthopedic component (10) can be fixed on the holder (50).

3. The supply system as claimed in claim 2, **characterized in that** the fastening device (40) comprises at least one form-fit element (42) and/or force-fit element, which cooperates with a correspondingly equipped form-fit element (41) or force-fit element on the orthopedic component (10).

4. The supply system as claimed in one of the preceding claims, **characterized in that** encoding (25), which can be read by means of an identification device (65, 80) arranged in on the holder (50), is stored in the orthopedic component (10), and/or **in that** an identification device (125), by means of which encoding (165) arranged in or on the holder (50) can be read, is arranged in the orthopedic component (10).

5. The supply system as claimed in one of the preceding claims, **characterized in that** a cleaning apparatus (70) is arranged on the holder (50).

6. The supply system as claimed in claim 5, **characterized in that** the cleaning apparatus (70) comprises a UV source, a plasma generator and/or an X-ray tube.

7. A system consisting of a supply system as claimed in one of the preceding claims and an orthopedic component.

8. A method for supplying an orthopedic component (10) with data and/or energy, the orthopedic component (10) comprising at least one electronic and/or electrical device (11, 12) and being equipped with a supply connection (20) and/or a radio device (31) for receiving data and/or electrical energy, whereas the orthopedic component (10) is fastened in or on a holder (50) and on a supply system according to one of the preceding claims, in or on which a supply device (30, 60), compatible with the supply connection (20) and/or the radio device (31), for supplying the orthopedic component (10) with data and/or energy is arranged, the supply connection (209 and/or the radio device (31) is coupled to the supply device (30, 60) and data and/or energy transmission takes place automatically, **characterized in that** after the coupling to the supply device (30, 60), a mode switchover of the orthopedic component (10) from an operating mode to a supply mode is carried out.

9. The method as claimed in claim 8, **characterized in that** identification of the orthopedic component (10) is carried out before the data and/or energy transmission.

10. The method as claimed in claim 9, **characterized in that**, as a function of the orthopedic component (10), data adapted to the respective orthopedic component (10) are transmitted or functional tests are instigated.

## Revendications

1. Système d'alimentation pour au moins un composant orthopédique (10) qui comprend au moins un dispositif électronique et/ou électrique (11, 12) et qui est équipé d'une connexion d'alimentation (20) et/ou d'un dispositif radio (31) destiné(e) à recevoir des données et/ou de l'énergie électrique, le système d'alimentation présentant un support (50) pour le composant orthopédique (10), qui comprend un dispositif d'alimentation (30, 60) compatible avec la connexion d'alimentation (20) et/ou avec le dispositif radio (31) et destiné à alimenter le composant orthopédique (10) en données et/ou en énergie, le dispositif d'alimentation (60) et la connexion d'alimentation (20) étant conçus comme des dispositifs de transmission sans contact pour la transmission inductive ou capacitive ou pour la transmission radio,
**caractérisé en ce que** le système d'alimentation est conçu pour effectuer, après un couplage du composant orthopédique (10) avec le dispositif d'alimentation (30, 60), un changement de mode du composant orthopédique (10) pour passer d'un mode de fonctionnement à un mode d'alimentation.

2. Système d'alimentation selon la revendication 1,
**caractérisé en ce que** le support (50) est réalisé sous forme de tablette ou de logement présentant au moins un dispositif de fixation (40) qui permet de fixer le composant orthopédique (10) au support (50).

3. Système d'alimentation selon la revendication 2,
**caractérisé en ce que** le dispositif de fixation (40) présente au moins un élément de liaison par coopération de forme (42) et/ou un élément de liaison par coopération de force qui interagit avec un élément de liaison par coopération forme (41) ou avec un élément de liaison par coopération de force, équipé de manière correspondante, sur le composant orthopédique (10).

4. Système d'alimentation selon l'une des revendications précédentes, **caractérisé en ce qu'**un codage (25) est déposé dans le composant orthopédique (10), qui peut être lu par un dispositif d'identification (65, 80) disposé dans ou sur le support (50), et/ou
**en ce qu'**un dispositif d'identification (125) est disposé dans le composant orthopédique (10), qui permet de lire un codage (165) disposé dans ou sur le support (50).

5. Système d'alimentation selon l'une des revendications précédentes, **caractérisé en ce qu'**un dispositif de nettoyage (70) est disposé sur le support (50).

6. Système d'alimentation selon la revendication 5,
**caractérisé en ce que** le dispositif de nettoyage (70) comprend une source UV, un générateur de plasma et/ou un tube à rayons X.

7. Système constitué d'un système d'alimentation selon l'une des revendications précédentes et d'un composant orthopédique.

8. Procédé d'alimentation d'un composant orthopédique (10) en données et/ou en énergie, le composant orthopédique (10) comprenant au moins un dispositif électronique et/ou électrique (11, 12) et étant équipé d'une connexion d'alimentation (20) et/ou d'un dispositif radio (31) destiné(e) à recevoir des données et/ou de l'énergie électrique,
dans lequel le composant orthopédique (10) est fixé dans ou sur un support (50) et sur un système d'alimentation selon l'une des revendications précédentes, dans ou sur lequel est disposé un dispositif d'alimentation (30, 31) compatible avec la connexion d'alimentation (20) et/ou avec le dispositif radio (31) et destiné à alimenter le composant orthopédique (10) en données et/ou en énergie,
la connexion d'alimentation (20) et/ou le dispositif radio (31) est couplé(e) au dispositif d'alimentation (30, 60), et une transmission de données et/ou d'énergie s'effectue automatiquement,
**caractérisé en ce qu'**après le couplage avec le dispositif d'alimentation (30, 60), un changement de mode du composant orthopédique (10) s'effectue pour passer d'un mode de fonctionnement à un mode d'alimentation.

9. Procédé selon la revendication 8,
**caractérisé en ce qu'**une identification du composant orthopédique (10) s'effectue avant la transmission de données et/ou d'énergie.

10. Procédé selon la revendication 9,
**caractérisé en ce que** des données adaptées au composant orthopédique (10) respectif sont transmises, ou des contrôles de fonctionnement sont initiés, en fonction du composant orthopédique (10).
